# FASCICULE DE BREVET EUROPEEN

(11) **EP 2 131 871 B1**
(45) Date de publication et mention de la délivrance du brevet: **08.01.2014**
(21) Numéro de dépôt: 08718148.3
(22) Date de dépôt: 21.03.2008
(51) Int. Cl.: A61K 49/18, B82Y 5/00

(54) **UTILISATION DE NANOPARTICULES METALLIQUES DANS LE DIAGNOSTIC DE LA MALADIE D'ALZHEIMER**
VERWENDUNG VON METALL-NANOTEILCHEN ZUR DIAGNOSE DER ALZHEIMER ERKRANKUNG
USE OF METAL NANOPARTICLES FOR DIAGNOSING ALZHEIMER DISEASE

(30) Priorité: 22.03.2007 FR 0753980
(43) Date de publication de la demande: 16.12.2009
(73) Titulaire: GUERBET, 93420 Villepinte (FR)
(72) Inventeur: COROT, Claire, F-69006 Lyon (FR)
(74) Mandataire: Regimbeau
(86) Numéro de dépôt international: PCT/EP2008/053450
(87) Numéro de publication internationale: WO 2008/125422

(56) Documents cités:
- WO-A-02/094191
- WO-A-2004/058275
- WO-A-2006/102377
- FR-A- 2 861 994
- MARTY B. ET AL: "Dynamic study of blood-brain barrier closure after its disruption using ultrasound: a quantitative analysis", JOURNAL OF CEREBRAL BLOOD FLOW & METABOLISM, 2012, pages 1-11,

## Description

L'invention concerne l'utilisation de nanoparticules métalliques, avantageusement revêtues d'une couche protectrice organique, pour la preparation d'une composition le diagnostic en IRM de la maladie d'Alzheimer.

La maladie d'Alzheimer (AD) est une maladie neurodégénérative caractérisée par une altération de la fonction cognitive associant troubles de la mémoire, de la fonction intellectuelle et de la personnalité sans modification de l'état de conscience. AD existe sous 2 formels: une forme tardive et sporadique, la plus courante, qui représente 90 à 95% des cas alors et une forme familiale (caractère autosomique dominante) qui ne représente que 5 à 10% de tous les cas connus. L'examen du cerveau de patients atteints de AD met en évidence des lésions histopathologiques bien précises qui sont les plaques séniles (SPs: seniles plaques) et les dégénérescences neurofibrillaires (NFTs: neurofibrillary tangles).

Les SPs sont très nombreuses dans le cortex cérébral des patients AD. Elles sont également trouvées dans la Trisomie 21 et à des taux variables, généralement faibles, au cours du vieillissement cérébral normal.

A l'échelle de la microscopie optique ce sont des dépôts extracellulaires ayant une structure sphérique microscopique formée d'un coeur de protéines insolubles (substance amyloïde) entouré de prolongements neuronaux dégénérés. A l'échelle de la microscopie électronique, la substance amyloïde est formée de faisceaux de filaments droits d'un diamètre de 6 à 9 nm. Ces filaments occupent le domaine extracellulaire du tissu nerveux central. A l'échelle moléculaire, ces amas protéiniques sont essentiellement constitués de la forme courte du peptide bêta-amyloïde (Aβ₄₀), filament polypeptidique adoptant une structure en feuillets lui conférant son caractère insoluble et probablement sa toxicité. Aβ est le produit du catabolisme d'un précurseur protéique membranaire, l'APP *(amyloid protein precursor).*

Les dégénérescences neurofibrillaires correspondent à l'accumulation de fibrilles pathologiques dans le cytoplasme des neurones. Il s'agit donc d'une lésion intraneuronale. Ce sont surtout les cellules pyramidales du cortex cérébral associatif et de la structure hippocampique qui sont touchées par ce phénomène.

L'art antérieur décrit des composés spécifiques vectorisés, notamment en imagerie de fluorescence, en IRM (Imagerie Résonance Magnétique) ou en scintigraphie, comprenant d'une part une entité signal et d'autre part un biovecteur (agent de ciblage) destiné au ciblage moléculaire spécifique d'un marqueur surexprimé dans la maladie d'Alzheimer, et en particulier de ciblage de plaques amyloïdes. Par exemple le document US2006051293 décrit des ligands de ciblage peptidiques et non peptidiques couplés à des chélates de métaux paramagnétiques ou de radionucléides.

La très grande majorité des publications concerne la médecine nucléaire, qui a l'avantage de la forte sensibilité mais l'inconvénient, en plus de son caractère irradiant, de la faible résolution par rapport à l'IRM. Le document WO02094191 décrit certes pour l'IRM des particules désignées MION (monocristallines), comprenant un noyau d'oxyde de fer, comprenant une couche de dextran et couplées à un biovecteur peptidique de ciblage de l'AD, mais il s'agit de peptides complexes à fabriquer et à coupler.

FR2861994 décrit des nanoparticules recouvertes d'un revêtement dépourvu de composé de ciblage spécifique de métalloprotéinases et constitué de dérivés polymériques ou non-polymériques, en particulier des branches PEG et aminoalcool, pour le diagnostic par IRM de maladies liés aux métalloprotéinases, telles que la maladie d'Alzheimer.

Parmi les différents problèmes techniques à résoudre, le demandeur a recherché des composés efficaces en imagerie IRM de la maladie d'Alzheimer, de type nanoparticules métalliques, différents de l'art antérieur, et notamment des nanoparticules métalliques non vectorisées par un biovecteur de ciblage spécifique, avec ainsi l'avantage d'une synthèse moins complexe et coûteuse que les produits vectorisés.

Pour cela, il a travaillé sur des nanoparticules présentant notamment une captation améliorée par les macrophages qui sont associés à un mécanisme d'inflammation corrélé à l'AD, et sont capables de traverser la BHE (barrière hémato encéphalique ou blood brain barrier). Le demandeur a aussi travaillé sur des nanoparticules capables de traverser la BHE au moins en partie directement, par un mécanisme biologique de transport passif ou actif, grâce à l'utilisation de groupes facilitant le transfert de la BHE décrits dans la demande.

Dans la demande, on utilise le terme captation macrophagique par souci de simplification, mais ce terme inclut le cas de la captation par tout système cellulaire du système immunitaire notamment phagocytaire associé à la maladie AD et impliqué dans un processus inflammatoire/immunitaire localisé en particulier au niveau des plaques amyloïdes ou d'autres territoires atteints du cerveau avec activation des cellules phagocytaires. Il s'agit plus. spécialement d'une captation par les monocytes, les macrophages, les microglies, ou tout autre type cellulaire connu de l'homme du métier.

L'invention concerne plus précisément selon un aspect l'utilisation de nanoparticules métalliques pour la préparation d'une composition de diagnostic en IRM pour le diagnostic de la maladie d'Alzheimer (AD), ces particules comprenant un noyau recouvert d'une couche non polymérique dépourvue de biovecteur de ciblage spécifique de l'AD. Cette couche permet de protéger le noyau.

Par l'expression « couche polymérique dépourvue de biovecteur de ciblage spécifique de l'AD », on entend que la couche polymérique et les nanoparticules ne présentent pas de ligands connus de l'art antérieur (notamment des peptides ou des anticorps) pour cibler spécifiquement des marqueurs de l'AD tels que les peptides amyloïdes.

Selon l'invention, sur la couche non polymérique dépourvue de biovecteur de ciblage sont greffés des groupes favorisant la captation macrophagique tels que les groupes hydrophiles décrits en détail ci après dans la demande, et/ou des groupes polymériques favorisant le transfert de la BHE. Toutefois ces groupes ne comprennent pas les biovecteurs connus de l'art antérieur pour reconnaître spécifiquement des récepteurs macrophagiques (récepteurs SRA notamment).

La couche non polymérique de la présente invention est anionique et est un gem-bisphosphonate de formule (II) telle que définie dans la revendication 1. On peut citer comme exemple de couches anioniques les couches suivantes (ainsi que leurs dérivés connus) qui ne font toutefois pas partie de la présente invention :
- phosphate, phosphonate, phosphonate monoester, diphosphonate, bisphosphonate, gem-bisphosphonate, diphosphate, thiophosphate, thiophosphonate, polyphosphate, phosphinate ;
- sulfonate, bisulfonate ;
- hydroxamate, arginine hydroxamate ;
- silane, ou dérivé de silice, silanetriol, siloxane, trialcoxysilane ;
- acide aminé ;
- mercapto, acide dimercaptosuccinique ;
- carboxylate, acide di ou polycarboxylique aliphatique (malique, citrique, tartrique, aspartique, gluconique notamment), avantageusement un acide aliphatique comportant au moins trois fonctions -COOH, acide cyclohexane-tricarboxylique, acide cyclohexanehexacarboxylique ;
- cathécolate.

Le tableau 1 ci après montre les exemples de structure de certaines couches non polymériques:

| Fonction | Structure |
|---|---|
| phosphonate | 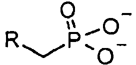 |
| Phosphonate monoester | 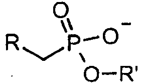 |
| phosphinate | 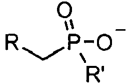 |
| Sulfonate | 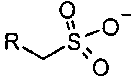 |
| hydroxamate | 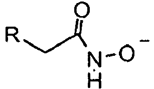 |
| Siloxane | 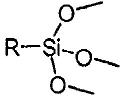 |
| catécholate | 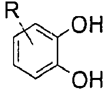 |

Dans ces structures :
R' représente un groupe alkyle linéaire ou ramifié, le cas échéant substitué.
R représente un groupe X1-L, dans lequel L représente un groupe de liaison (par exemple alkyle, linéaire ou ramifié, le cas échéant substitué), et X1 représente une fonction capable d'assurer le couplage (c'est-à-dire le greffage) de la couche non polymérique avec des groupes favorisant la captation macrophagique et/ou des groupes facilitant le transfert de la BHE.

L'homme du métier comprend clairement que les groupements oxygénés du tableau ci-dessus permettent la fixation de la couche non polymérique au noyau métallique de la nanoparticule selon l'invention tandis que les groupes R sont destinés au couplage avec les groupes favorisant la captation macrophagique et/ou des groupes facilitant le transfert de la BHE.

Avantageusement L a la définition indiqué ci-dessous. De façon avantageuse X1 a la même définition que celle de X indiquée ci-dessous.

Selon l'invention, la couche non polymérique est la couche *gem*-bisphosphonate de formule (II) :
X-L-CH(PO₃H₂)₂ décrite dans WO 2004/058275 dans laquelle :
   - L représente un groupe organique de liaison reliant la fonction X à la fonction *gem-*bisphosphonate -CH(PO₃H₂)₂ ;
   - X représente une fonction chimique telle que définie plus bas.
L représente un groupement divalent choisi parmi :
   - un groupe aliphatique (par exemple en C₁ à C₅) ; alicyclique ; alicyclique aliphatique ; aromatique ; aromatique aliphatique, lesdits groupes aliphatiques, alicycliques et aromatiques pouvant être éventuellement substitués par un groupe méthyle, hydroxy, méthoxy, acétoxy, amido, ou un atome d'halogène, avantageusement un atome de chlore, d'iode ou de brome ;
   - un groupement -L₁-NHCO-L₂ où L₁ et L₂ identiques ou différents représentent un groupe aliphatique ; alicyclique ; aromatique ; alicyclique aliphatique ou aromatique aliphatique, lesdits groupes pouvant être éventuellement substitués par un groupe méthyle, hydroxy, méthoxy, acétoxy, amido ou un atome d'halogène, avantageusement un atome de chlore, d'iode ou de brome.
X représente un groupe -COOH, -NH₂, -NCS, -NH-NH₂, -CHO, alkylpyrocarbonyle (-CO-O-CO-alk), acylazidyle (-CO-N₃), iminocarbonate (-O-C(NH)-NH₂), vinylsulfuryle (-S-CH=CH₂), pyridyldisulfuryle (-S-S-Py), haloacétyle, maléimidyle, dichloro-triazinyle, halogène, les groupes -COOH et -NH₂ étant particulièrement préférés.

La couche non polymérique est telle que des groupes favorisant la captation macrophagique groupes le de la et/ou des groupes facilitant le transfert de la BHE tels que définis dans la revendication 1, sont greffés sur cette couche non polymérique. Ainsi sur la couche non polymérique sont greffés :
- soit des groupes favorisant la captation macrophagique,
- soit des groupes facilitant le transfert de la BHE,
- soit à la fois des groupes favorisant la captation macrophagique et des groupes facilitant le transfert de la BHE.

Par l'expression couche non polymérique, on entend des couches non polymériques telles que par exemple celles citées ci-dessus, différentes des couches polymériques connues et décrites plus loin (polysaccharidiques en particulier). Avantageusement une couche non polymérique ne comprend aucun groupe polymérique. Toutefois, une couche non polymérique peut comprendre quelques groupes polymériques, PEG, capables de se lier directement au noyau métallique. Dans ce cas, une telle couche comprend moins de 50% en poids de groupes polymériques PEG, avantageusement entre 1 et 25% en poids, de façon encore plus avantageuse entre 1 et 20% en poids, de façon particulièrement avantageuse, entre 1 et 15% en poids de groupes polymériques PEG. Ces groupes polymériques ne sont pas des polysaccharides.

On décrit maintenant des variantes de groupes favorisant la captation macrophagique (appelés groupes de captation) et/ou des groupes facilitant le transfert de la BHE (appelés groupes de transfert).

Les groupes de captation de la présente invention (qui peuvent aussi le cas échéant favoriser le transfert de le BHE) sont des groupes hydrophiles.

Ces groupes hydrophiles ont un poids moléculaire (masse molaire en g/mol) supérieur à 200, et sont sélectionnés parmi:
2) le groupe amino-alcool de formule :

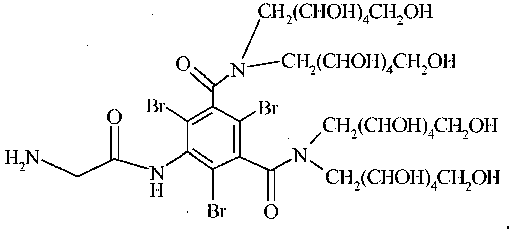

Selon l'invention, les groupes de transfert (susceptibles de favoriser le cas échéant aussi la captation macrophagique) sont des groupes polymériques polyéthylène glycol (PEG) et ses monoéthers et monoesters en C1 à C3, et les amino-PEG, de masse moléculaire de préférence de 350 à 2000. Il ne s'agit pas de polysaccharides.

Parmi les groupes PEG on pourra utiliser notamment des groupes (CH₂CH₂O)_{q}(CH₂)ᵣ-CO- , (CH₂CH₂O)_{q}(CH₂)ᵣ-NH-CO- avec q = 1-10 et r=2-10 ; par exemple PEG 300, PEG 700, PEG 750, PEG 1000, PEG 1500, PEG 2000.

Le greffage des groupes de captation et des groupes de transfert a lieu par liaison covalente avec des groupes de la couche non polymérique par des procédés bien connus de l'homme du métier. Par exemple dans le cas des groupes polymériques amino PEG et des couches de formule Il dans lesquelles X représente un groupe -COOH, la fonction amine des groupes amino PEG est couplée à la fonction acide du composé (II).

Le taux de greffage et la nature des groupes de captation et des groupes de transfert sont déterminés en fonction des propriétés biologiques et chimiques recherchées.

Selon une réalisation, ce taux de greffage est inférieur à 50% en poids et avantageusement compris entre 1 et 25% en poids, de façon encore plus avantageuse entre 1 et 20% en poids, de façon particulièrement avantageuse, entre 1 et 15% en poids.

Selon une autre réalisation, ce taux de greffage est supérieur à 50%, avantageusement compris entre 70 et 95%, de façon encore plus avantageuse entre 80 et 90%.

La nanoparticule présente avantageusement un taux de greffage en groupes de captation compris entre 10 et 100 %, avantageusement entre 50 et 95%, entre 60 et 90%, par exemple de l'ordre de 60,70, 80, 90%. Par exemple, 1, 3, 5, 10, 20, 25, 50, 75, 80, 90, 95 % de ses groupes X (par exemple acides) sont couplés à un groupe de captation.

La nanoparticule présente avantageusement selon d'autres réalisations un taux de greffage en groupes de transfert compris entre 10 et 100 %, avantageusement entre 50 et 95%, entre 60 et 90%, par exemple de l'ordre de 60,70, 80, 90%. Par exemple, 1, 3, 5, 10, 20, 25, 50, 75, 80, 90, 95 % de ses groupes X (par exemple acides) sont couplés à un groupe de transfert. Le taux et la longueur des groupes de transfert (en particulier des PEG) sont adaptés pour éviter de trop masquer l'UPSIO, le rendant sinon trop furtif vis-à-vis des cellules phagocytaires.

Selon des réalisations avantageuses, on utilisera :
- soit des groupes hydrophiles favorisant la captation correspondant à des amino alcools tels que décrits précédemment et dans la revendication 1, à un taux de greffage compris entre 10 et 100 %, avantageusement entre 50 et 95%, entre 60 et 90%, par exemple 60,70, 80, 90%
- des groupes PEG favorisant le transfert à un taux de greffage inférieur à 50%, avantageusement inférieur à 25%, par exemple de 0 à 10%, par exemple 5 à 10%,
- soit à la fois des groupes hydrophiles amino-alcools tels que définis dans la revendication 1 et des groupes PEG.

Selon des réalisations avantageuses, on greffera d'une part des groupes de captation (avantageusement entre 50 et 95% des sites de greffage possibles sur la particule), et d'autre part des groupes de transfert (avantageusement entre 10 et 30 % des sites de greffage possibles sur la particule). L'homme du métier comprend que le taux de greffage total (c'est-à-dire le taux de greffage des groupes captation + le taux de greffage des groupes de transfert) n'est pas nécessairement de 100%, la couche non polymérique pouvant présenter des fonctions susceptibles d'être greffées mais ne l'étant pas.

Dans le tableau 2 ci après sont rassemblés des exemples dans lesquels on indique le taux de greffage (en %) des groupes aminoalcools tels que définis dans la revendication 1 et des dérivés PEG ainsi que le % de fonctions susceptibles d'être greffées mais ne l'étant pas.

| | Ex 1 | Ex 2 | Ex 3 | Ex 4 | Ex 5 | Ex 6 |
|---|---|---|---|---|---|---|
| Taux de greffage des groupes amino alcool | 95 | 60 | 40 | 80 | 70 | 20 |
| Taux de greffage des dérivés PEG | 5 | 20 | 20 | 20 | 30 | 60 |
| % de fonctions susceptibles d'être greffées mais ne l'étant pas | 0 | 20 | 40 | 0 | 0 | 20 |

Certains exemples du tableau 1 sont avantageux en particulier pour le diagnostic de AD lorsque l'état inflammatoire est déjà assez avancé : la BHE laisse passer un nombre accru de macrophages et l'USPIO traverse la BHE au moins en partie par l'intermédiaire de cellules du système immunitaire, ce qui permet d'utiliser un taux de greffage des groupes de captation supérieur (80% par exemple).

Pour un ciblage plutôt précoce, on pourra préférer augmenter la part de groupes de transfert (PEG notamment) pour faciliter l'accès des USPIO jusqu'aux sites d'inflammation du cerveau, notamment au niveau des plaques amyloïdes. On pourra alors utiliser par exemple un taux de greffage de PEG compris entre 40 et 80%.

Pour le ciblage précoce, on pourra aussi utiliser des taux de greffage de groupes de captation élevés dans le cas où l'on aura facilité l'ouverture de le BHE (choc au mannitol par exemple), ou lorsque les USPIO sont encapsulés dans des vecteurs de transfert décrits plus loin.

Le mécanisme de transfert des USPIO porteurs de groupes de transfert n'est pas totalement élucidé. Le demandeur fait l'hypothèse que ce transfert fait intervenir un mécanisme biologique de transport actif et/ou passif, via des vésicules par exemple, ou par un phénomène d'opsonisation.

Dans des réalisations, le demandeur a réussi à obtenir des produits capables d'être captés par des cellules du système immunitaires (macrophages en particulier), grâce à leurs propriétés physico-chimiques (état de charge, reconnaissance par les opsonines ...), tout en étant capables de traverser la BHE. En particulier, les groupes PEG ont été choisis de manière que ces USPIO greffés ne soient pas trop furtifs pour être captés par les macrophages.

On décrit maintenant plus précisément la structure du noyau métallique.

Selon un mode de réalisation, le noyau est monocristallin, de préférence à base d'un composé du fer. On a aussi étudié d'autres noyaux tels que l'or ou le tungstène.

On rappelle que les particules à base d'un composé du fer comprennent avantageusement du fer (III), généralement un oxyde ou un hydroxyde de fer. Le noyau des ces particules magnétiques est composé typiquement en tout ou partie d'hydroxyde de fer ; d'oxyde de fer hydraté ; de ferrites ; d'oxydes de fer mixtes tels que des oxydes de fer mixtes de cobalt, de nickel, de manganèse, de béryllium, de magnésium, de calcium, de baryum, de strontium, de cuivre, de zinc ou de platine; ou d'un mélange de ceux-ci. Le terme de "ferrite" désigne les oxydes de fer de formule générale [x Fe₂O₃, y MO_{z}], où M désigne un métal magnétisable sous l'effet d'un champ magnétique tel que Fe, Co, Ru, Mg, Mn, le métal magnétisable pouvant être éventuellement radioactif.

De façon avantageuse, les particules magnétiques des compositions utilisées dans le cadre de l'invention comprennent une ferrite, notamment la maghémite (y Fe₂O₃) et la magnétite (Fe₃O₄), des ferrites mixtes de cobalt (Fe₂CoO₄) ou de manganèse (Fe₂MnO₄).

Selon des réalisations, les nanoparticules comprennent un noyau polycristallin recouvert de la couche non polymérique. Par exemple le noyau comprend plusieurs cristaux d'oxydes de fer différents.

Selon un autre mode de réalisation, les nanoparticules comprennent un noyau obtenu à partir de métal non supeparamagnétique, notamment à partir d'oxyde de lanthanide comme le gadolinium ou l'europium, ou à partir d'autres cristaux mixtes dont l'obtention est décrite en détail par exemple dans WO2006/031190.

Les nanoparticules ont une taille typiquement comprise entre 2 et 200 nm, avantageusement entre 10 et 60 mn, avantageusement de l'ordre de 5, 10, 20, 30, 40, 50 ou 60 nm. La couche non polymérique forme un enrobage du noyau.

Selon un autre aspect, les nanoparticules recouvertes d'une couche non polymérique dépourvue de biovecteur de ciblage spécifique de l'AD, mais portant des groupes de captation et/ou des groupes de transfert, sont associés à (couplées à et/ou incorporés dans, avantageusement incorporées dans) un vecteur de transfert de la BHE, ce vecteur de transfert étant destiné à permettre et/ou augmenter passage de la BHE par les nanoparticules.

Parmi les vecteurs de transfert de la BHE on citera :
- des vecteurs lipidiques tels que des liposomes, des systèmes d'émulsions, des micelles, des systèmes mixtes liposomes-micelles
- des surfactants appropriés, aptes à former un enrobage ou une capsule protectrice autour de la nanoparticule métallique, par exemple choisis parmi les suivants : esters d'acides gras et glycérol, sorbitol et autres alcools multifonctionnels, glycérol monostéarate, sorbitan monolaurate, sorbitan monoléate, poloxamines, éthers de polyoxyéthyléne et esters de polyoxyéthylène, triglycérides éthoxylés, phénols et diphénols éthoxylés, surfactant du Genapol, des sels métalliques d'acides gras, lauryle sulfate de sodium, dodécylsulfate de sodium, sels métalliques de sulfosuccinates, polysorbates, poloxamer, polyoxyéthylène glycols, polybutylcyanoacrylate, tween, homopolymére polylactique (PLA) et/ou hétéropolymère poly(lactique-co-glycolique) (PLGA)
- des macromolécules de transport telles que des protéines en particulier des lipoprotéines

Pour les liposomes on utilisera par exemple des sphingolipides, glycolipides, glycérolipides, phospholipides, du cholestérol. On utilisera par exemple les composés suivants : phosphatidique acide, phosphatidyl choline, phosphatidylsérine, phosphatidylglycérol, phosphatidyléthanolamine, phosphatidylinositol, cardiolipide ; des phospholipides neutres comme la dipalmitoyl phosphatidylcholine (DPPC), dimyristoyl phosphatisylcholine (DMPC), dilauroyl phosphatidylcholine (DLPC), dioleyl phosphatidylcholine (DOPC), phosphatidylcholine (PC), distearoyl phosphatidylcholine (DSPC), sphingomyeline (SM), monosialoganglioside, sulfogalactosylceramide. On peut avantageusement utiliser des liposomes pégylés.

La préparation de liposomes est connue de l'art antérieur et est par exemple décrite dans US5643599, WO2006115416 par la technique habituelle avec film d'hydratation. De tels documents d'écrivant la préparation de liposomes sont incorporés par référence.

Selon des variantes, les vecteurs de transfert de la BHE sont capables de traverser la BHE au moins en partie via des récepteurs lipidiques.

Avantageusement les vecteurs de transfert de la BHE sont aptes à libérer les nanoparticules selon l'invention une fois la BHE franchie, le cas échéant après activation par exemple chimique ou physique (température, ultrasons ...). On utilisera par exemple des liposomes thermosensibles capables de libérer les USPIO et éventuellement un principe actif thérapeutique également inclus dans le liposome.

Selon un autre aspect, le vecteur de transfert de la BHE utilisable dans le cadre de la présente invention comprend, avantageusement est porteur de, un biovecteur de ciblage de l'AD, avantageusement un biovecteur de ciblage spécifique de l'AD. Parmi les biovecteurs de ciblage d'AD possibles on utilisera des benzothiazoles, les peptides (Ass,Apl par exemple) de WO 2094191. On citera également des biovecteurs de ciblage de l'AD décrits dans l'art antérieur en diagnostique ou en thérapeutique, par exemple : benzodiazépines, thiophènes, furanes, pyrrole, pyrazole, ligands limitant la formation de peptides Abeta, anticorps anti-Abeta, ligands interagissant avec des récepteurs glutamate tels que des sulfanamides, des composés de type 1-aminocyclohexane, des inhibiteurs de secrétase ; d'autres biovecteurs identifiés comme modulant un mécanisme enzymatique et/ou de synthèse lié à l'AD par exemple de ciblage de secrétases ; d'autres biovecteurs identifiés comme capable de moduler ou de suivre l'activité de neurotransmetteurs.

On peut ainsi utiliser aussi des biovecteurs identifiés (peptides notamment de ciblage des plaques ou des NFT ou de précurseurs identifiés de ces plaques et NFT) comme aptes à une reconnaissance précoce de l'AD, y compris le cas échant ceux qui jusqu'à présent n'étaient pas retenus pour le diagnostique de l'AD en raison de leur difficulté ou incapacité à traverser la BHE en l'absence de ce vecteur de transfert.

Le cas échéant, la composition de diagnostic selon l'invention comprend, outre les nanoparticules selon l'invention détectables en imagerie diagnostique, au moins un agent thérapeutique contre l'AD. En particulier dans le cas des nanoparticules associées à un vecteur de transfert de la BHE, l'agent thérapeutique contre l'AD est associé audit vecteur de transfert de la BHE. Avantageusement il est incorporé dans le vecteur de transfert d la BHE.

Selon des réalisations, la composition diagnostique selon l'invention comprend en outre un agent facilitant la passage de la BHE, tel que le mannitol ou d'autres composés appropriés, en particulier lorsque la composition diagnostique est administrée à un stade peu avancé de la maladie AD.

Selon un autre aspect l'invention concerne l'imagerie multimodale utilisant des compositions décrites ci-dessus pour l'IRM, combinée à d'autres modalités d'imagerie telles que avantageusement par exemple la MPI, l'imagerie PET avec des composés connus pour l'imagerie PET de la maladie d'Alzheimer (par exemple un biomarqueur ligand de plaques amyloïdes couplé à un chélate marqué au technicium, gallium ou tout autre radionucléide), le scanner aux rayons X, l'imagerie IRM avec des gadofluorines, l'imagerie optique, l'imagerie de fluorescence, l'imagerie SPECT, notamment de perfusion. De plus l'imagerie peut être associée à différentes modalités de cartographie cérébrale, CBV (cerebral blood volume) par exemple. Toutes ces différentes modalités comprennent typiquement des méthodes et systèmes de traitement d'image, analysant et le cas échéant régulant l'administration de produit de contraste ou de traitement thérapeutique en fonction de données du patient, de bases de données sur des patients AD.

Parmi les différentes sous-indications diagnostiques du diagnostic de la maladie d'Alzheimer, on citera non seulement le diagnostic au stade avancé (typiquement avec inflammation), mais aussi à un stade précoce ou intermédiaire de manière telle qu'elles permettent d'établir ou de confirmer un niveau de risque pathologique, et le cas échéant de suivre l'efficacité d'un traitement thérapeutique précoce et/ou tardif contre la maladie (médicament ou candidat médicament encore au stade préclinique ou au stade des essais cliniques). Le demandeur a en effet identifié la captation macrophagique comme moyen d'accès aux zones pathologiques mais il est possible que d'autres mécanismes permettent ou facilitent le passage de la BHE par les nanoparticules décrites.

Selon un autre aspect, l'invention concerne l'utilisation des nanoparticules citées dans la demande pour la préparation d'une composition de diagnostic destinée au suivi diagnostique de l'efficacité d'un médicament ou d'un candidat médicament contre la maladie d'Alzheimer (et une méthode de diagnostic ou de suivi diagnostique d'un traitement thérapeutique utilisant les nanoparticules ci-dessus).

En ce qui concerne plus spécifiquement l'utilisation des composés décrits pour le suivi de traitement thérapeutique de l'AD, on administre, dans une même composition ou dans des compositions séparées, de manière simultanée ou différée dans le temps, d'une part au moins un médicament thérapeutique ou agent thérapeutique contre la maladie d'Alzheimer, et d'autre part au moins un agent de contraste de la demande.

Avantageusement l'agent de contraste permet d'identifier rapidement l'efficacité du traitement, et permet de mieux cibler le traitement thérapeutique le plus adapté. Par exemple, le traitement thérapeutique comprend au début une étape d'administration du médicament thérapeutique, et de l'agent de diagnostique (USPIO du demandeur), puis en fonction du premier résultat obtenu, l'orientation de la thérapie qui suit en continuant ce traitement ou en choisissant un autre traitement (plus économique et/ou efficace compte tenu de la base des données de résultats obtenus dans des cas similaires).

Les composés thérapeutiques (médicament ou agent thérapeutique) concernés sont tous ceux connus pour le traitement de l'AD, et sont avantageusement choisis parmi la liste qui suit (les références de brevets entre parenthèses sont des exemples de composés):
- modulateurs d'un mécanisme enzymatique (secrétases ...) et/ou de transduction et/ou modulateur d'une voie de signalisation connu de la maladie AD
- composés capables de moduler l'élaboration cellulaire de la protéine précurseur du bêta-amyloïde (APP) et pour la prévention ou le traitement de maladies associées à une élaboration anormale d'APP (WO2007132292)
- peptides capables de se lier à des plaques amyloïdes (WO2007145589)
- cytokines anti-inflammatoires (WO2007139178)
- dérivés isoxazole (WO2007137954)
- dérivés pyrazinone (WO2007135131)
- pyridazines et autres inhibiteurs de secrétase (et inhibiteurs de choline estérase) qui sont destinés à la modulation de voies cellulaires (par exemple de voies de transduction du signal) (WO2007130383)
- sulfonamides inhibiteurs de gamma secrétase (WO2007143523)
- pipéridines modulateurs de la gamma-sécrétase (WO2007125364)
- polypeptides de liaison préséniline/protéineG/c-src, intervenant dans des mécanismes de signalisation impliqués dans la maladie d'Alzheimer (WO2007123680)
- inhibiteurs d'acétylcholine estérase (WO2007122274)
- 2-AMINOPYRIMIDIN-4-ONES (WO2007114771)
- dérivés amides ou esters d'hydroxyéthylamine qui servent d'inhibiteurs efficaces de la béta-sécrétase (W02007110727)
- antagonistes du récepteur Histamine 3 (tétralines par exemple) (WO2007105053)
- inhibiteurs de récepteurs Apolipoprotéine E (WO2007098417)
- dérivés d'indazole (WO2007088401)
- régulateurs de la protéine ADAMTS4 (WO2007088399)
- antagoniste du récepteur couplé à la protéine G
- antagonistes du récepteur du glutamate métabotropique (mGluR)
- inhibiteurs de secrétase FLUORO SUBSTITUTED 2-OXO AZEPAN (W02007020190)
- dérivés malonamide (W02007110335)
- dérivés TETRALINE et INDANE antagonistes de 5-HT6 et/ou 5-HT2A (WO2006066790)
- 2-imidazoles (WO2007090720)
- ARYLSULFONYL BENZODIOXANES pour la modulation de récepteurs 5-HT6 et 5-HT2A (WO2005105776)
- Dérivés acide phosphinique inhibiteurs de beta-secretase (WO2005044830)
- TETRAHYDROCARBAZOLES et dérivés pour le traitement de maladies associées à des agonistes LXRalpha and/or LXRss (WO2005092856)
- DIBENZO-AZEPINE et BENZO-DIAZEPINE substitués inhibiteurs de GAMMA-SECRETASE (WO2005040126)
- Dérivés benzoxazepinone (WO2004100958)
- Dérivés THIAZOLOPYRIDINE ligands de récepteurs ADENOSINE (WO2005028484)
- antagonistes de récepteurs histamine H3 : ETHER HISTAMINE-3 (WO2007138431), AZABICYCLIC TETRALINES (WO2007105053), BENZIMIDAZOLE (WO2007069053), imidazoles inhibant la production de peptide Ass (WO2007034326)
- composés THIAZOLE SULFONAMIDE inhibiteurs de ma production de peptide Abeta (W02005097114)
- thiazole amines (WO2005095367,WO2005095368), oxazoles amine (WO2005095365), pyrazole aniline (WO2005095348), isothiazole et isoxazole amine (WO2005095361)
- tout composé contre l'inflammation dans la maladie d'Alzheimer, par exemple des agents inhibiteurs de COX-2 par exemple des sulfonyl pyrazoles par exemple des heterocyclo alkylsulfonyl pyrazoles (W003037351)
- composés cyclosporine (WO2006005580)
- tout agent ayant une activité sur des protéines kinases VEGFR-2, Tie-2, c-Src, c-Met, FGFR-1, Flt-1, HER-2, c-Abl, c-Raf, PDGFR-beta, ayant un effet contre des plaques amyloïdes, par exemple des composés PYRIDINYL-PYRIMIDINYLAMINO-BENZAMIDE (WO2005039586)
- vaccin anti beta amyloide (W02005014041)
- tout composé de ciblage modulateur de récepteurs GABA, notamment les composés suivants :
   - dérivés aryl-4-ethynyl-isoxazole à affinité et sélectivité pour les sites récepteurs GABA A a5 (W02007137954)
   - aryl-isoxazol-4-yl-imidazo[1,2-a]pyridine derivatives (WO2007082806)
   - dérivés isoxazol-4-yl-oxadiazole (WO2007071598)
   - dérivés imidazo benzodiazépine ; imidazo[1,5-a][1,2,4]triazolo[1,5-d][1,4]benzodiazepine (WO2007042421, WO2006045430)

Le diamètre hydrodynamique moyen de la structure de base des USPIO utilisées (coeur d'oxyde de fer recouvert de la couche) en solution est typiquement compris entre 2 et 500 nm. Avantageusement, au moins 90% des particules présentes, et de préférence au moins de 95% des particules présentes sont des particules individualisées, c'est à dire non agglomérées avec une ou plusieurs autres particules. Le diamètre hydrodynamique moyen auquel il est fait référence ici est le diamètre hydrodynamique moyen tel que mesuré par spectroscopie de corrélation de photons, par exemple par un appareil de type Zetasizer.

Les relaxivités r1 et r2 d'un produit de contraste magnétique donnent la mesure de son efficacité magnétique et permettent d'apprécier son influence sur le signal enregistré. Les composés obtenus présentent des relaxivités r1 et r2 avantageuses, permettant d'obtenir une forte augmentation des vitesses de relaxation des protons (RI = 1/T1 et R2 = 1/T2). Cet effet sur les vitesses de relaxation permet alors d'obtenir un bon contraste en IRM, dans les zones ciblées. La relaxivité r1 est de l'ordre de 10 à 50 mMol-ls⁻¹ et la relaxivité r2 de l'ordre de 20 à 400 mMol-ls⁻¹, à 20 Mhz. La teneur en fer de la particule (% en poids) est de l'ordre de 20 à 60%, typiquement de 30 à 50%.

La composition de diagnostic administrée comprend typiquement un type de nanoparticules mais on pourra aussi utiliser en association plusieurs nanoparticules différentes, de manière simultanée ou différée dans le temps.

La présente demande fournit des exemples de modèles permettant de tester l'efficacité diagnostique du composé comprenant des nanoparticules.

Les USPIO sont typiquement utilisées à une dose typiquement de 0,001 mol/kg à 10 mmol/kg en métal, par exemple de 1 mol/kg à 5 mmol/kg, par injection ou perfusion dans une artère ou une veine.

Les USPIO sont typiquement sous forme de solutions colloïdales stables (ou de suspensions de particules stabilisées) et peuvent être formulées sous forme de poudres lyophilisées à associer à un solvant approprié. Leur voie d'administration est connue de l'homme du métier, typiquement intraveineuse.

Les compositions de l'invention sont préférablement administrées par voie parentérale, les autres voies d'administration n'étant cependant pas exclues, l'administration sous forme d'une injection intraveineuse étant particulièrement préférée. L'injection est typiquement en bolus i.v, le cas échéant en intra-carotide, avec si nécessaire un choc osmotique au mannitol pour favoriser l'ouverture de la BHE. On pourra aussi réaliser une injection en intracérébral.

Les formes pour l'administration parentérale sont obtenues de façon conventionnelle par mélange des particules magnétiques avec des tampons, des agents stabilisants, des conservateurs, des agents solubilisants, des agents isotoniques et des agents de mise en suspension. Conformément aux techniques connues; ces mélanges sont ensuite stérilisés puis conditionnés sous la forme d'injections intraveineuses ou de lyophilisats prêts à être reconstitués dans un véhicule stérile pharmaceutiquement acceptable.

Les doses unitaires seront fonction de la composition des particules magnétiques, de la voie d'administration, du type de diagnostic à établir, ainsi que du patient. Les doses unitaires seront en général de 1-10 mmol de fer pour un homme de taille moyenne (75 kg). Parmi les adjuvants pharmaceutiques on pourra citer des agents conservateurs, stabilisateurs de pH, des antioxydants.

Il est intéressant de constater que de tels composés de la demande avec couche anionique ou polymérique sont également utilisables pour des indications différentes d'AD, telles que des maladies cardiovasculaires (athérome, athérosclérose...) ou inflammatoires ou cancéreuses.

Par ailleurs, le demandeur a étudié la comparaison pour le diagnostic de l'AD, de particules obtenues avec des nanoparticules comprenant un noyau monocristallin recouvert d'une couche polymérique, avantageusement polysaccharidique ou polymérique non saccharidique ; le polysaccharide étant en particulier choisi parmi le dextran et ses dérivés connus, l'amidon (et ses dérivés connus, par exemple hydroxyéthyle, hydroxyméthyle) ; le polymère non saccharidique étant en particulier un polymère ou un copolymère choisi parmi : les polyalkylène glycol notamment le polyéthylène glycol, polypropylène glycol, le polyglycérol, le polyoxyéthylène, les dérivés stéarate, les polyalkylcyanoacrylates, les composés polyvinyle, les cyclodextrines, les pectines, les glycosaminoglycans, les dérivés de cellulose et l'héparine, et les dérivés de ces divers polymères ou copolymères. Les nanoparticules peuvent comporter plusieurs couches polymériques successives.

On citera en particulier comme particule intéressante pour le diagnostic de l'AD des nanoparticules décrites dans WO2006412201, WO2006/031190, US2005/0260137, WO2004/107368.

Le demandeur a aussi étudié à titre comparatif l'utilisation de nanoparticules recouvertes d'une couche polymérique ou non polymérique citées dans la demande ci-dessus, et couplées à au moins un biovecteur de ciblage spécifique de l'AD (par exemple peptide ou anticorps tel qu'indiqué ci-dessus), ces nanoparticules étant associées à (incorporées dans ou couplées avec) un vecteur de transfert de la BHE tels que ceux indiqués ci-dessus. Les produits ainsi obtenus sont particulièrement intéressants lorsque l'on utilise un biovectcur de ciblage capable de détecter de préférence de manière précoce l'AD.

Le demandeur a ainsi étudié à titre comparatif le greffage à ces nanoparticules des groupes chimiques de type biovecteurs (peptides par exemple) capables de cibler des zones atteintes de maladie d'Alzheimer, ces biovecteurs étant ou non destinés à faciliter le passage de la BHE.

Le demandeur a étudié aussi à titre comparatif des composés comprenant greffées aux nanoparticules d'une part au moins un biovecteur de ciblage de zones atteintes et d'autre part au moins un ligand de passage de la BHE tel qu'un peptide ou autre composé approprié.

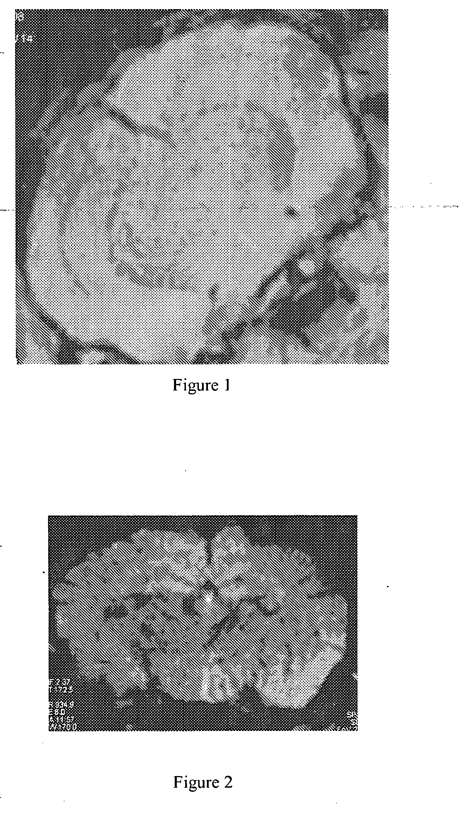

La description détaillé qui suit illustre des réalisations détaillées de composés et des résultats pour le diagnostic de la maladie d'Alzheimer
La figure 1 représente un exemple d'image RMN *in vivo* pré injection.
La figure 2 représente un exemple d'images RMN *in vivo* post injection obtenues 36h après injection du composé selon l'exemple I en utilisant le protocole selon l'exemple IV.

### I. Exemple de nanoparticule recouverte d'une couche non polymérique : couche gem-bisphosphonate avec branche hydrophile AAG₁AA₂₈Br (groupe de captation macrophagique).

Ce composé est obtenu comme à l'exemple 16 de WO 2004/058275. Sa formule est la suivante :

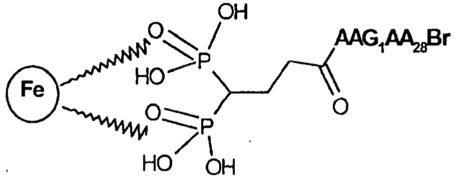

Son procédé de préparation est donc le suivant :
0,853 g (7,6 10⁻⁴ mole) du composé E de l'exemple 5 de WO2004/058275 sont dissous dans 13,55 ml de l'exemple 11 de WO2004/058275 à 0,279 M/L. Le pH est ajusté à 6,2.
171 mg de chlorhydrate de 1-(3-diméthylaminopropyl)-3-éthyl-carbodiimide sont ajoutés. L'ensemble est agité à température ambiante pendant 24 heures. La solution est ultrafiltrée sur une cellule à agitation PALL^{®} ayant un seuil de coupure de 30 kD. 500 ml de filtrat sont éliminés. Le rétentat est ajusté à 30 ml.

Le composé E de l'exemple 5 de WO2004/058275 : (N-N'-[bis(2,3,4,5,6-pentahydroxyhexyl)] 2,4,6-tribromo 5-(glycylamino)isophtlamide, désigné AAG₁AA₂₈Br (AA signifiant amino-alcool) a la formule suivante : 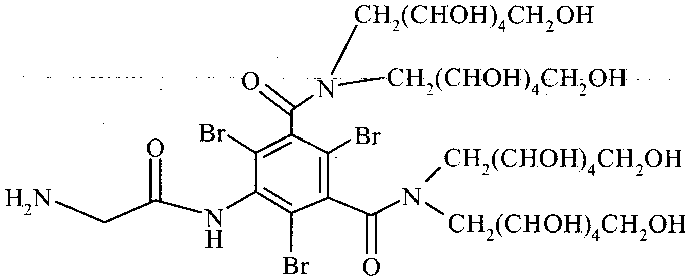 Il est préparé selon le mode opératoire décrit dans EP 0 922 700 A1.

La solution de l'exemple 11 de WO2004/058275 est préparée de la façon suivante: 50 ml de l'exemple 8 de WO2004/058275 à 4,73 M/L sont dilués dans 3 litres d'eau. Une solution de 1,3 g (5,24 10⁻³ mole) du composé A de l'exemple 1 de WO2004/058275 dans 80 ml d'eau est introduite goutte à goutte. La formule du composé A de WO2004/058275 est la suivante :

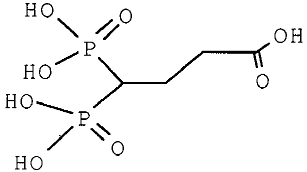

L'agitation est maintenue 30 min. Le floculat est isolé par décantation magnétique puis est lavé 3 fois par 3 litres d'eau. Il est remis en solution avec 700 ml d'eau à pH 11 avec QSP de NaOH [1N] puis stabilisé à pH 7,2 avec QSP de HCl [1N]. La solution finale est filtrée sur membrane 0,22 µm.

Les caractéristiques du composé de l'exemple 16 de WO2004/058275 sont les suivantes :
[Fe] = 0,132 M/L Taille PCS = 41,6 nm Poly σ = 0,22
Fe = 52,3 % Masse/Masse ; P = 0,77 % Masse/Masse ; C = 5,86 % Masse/Masse ; Br = 2,65 % Masse/Masse ;
Taux de greffage [composé A / Fe] = 1,33 % mole/mole
Taux de greffage [composé E / Fe] = 1,18% mole/mole
Taux de greffage [composé E / composé A] = 89 %

### II. Mise en évidence de la captation par les macrophages des nanoparticules obtenues à l'exemple I

Le composé gem-bisphosphonate répond positivement au test de phagocytose sur cellules THP1 (macrophages) activées, avec une captation inattendue de l'ordre de deux fois celle du Sinerem® (20 µg Fer/ 10⁷ C, au lieu de 8µg Fer/ 10⁷ C pour le Sinerem®), montrée à l'aide du test suivant.

### Protocole

- Ensemencer des flacons de 225cm² avec des THP-1.

### J0

- Préparer 110mL RPMI+10%**SVFi** + 122µL de **PMA** 10⁻⁴ M soit 25nM en final.
- Numérer les cellules THP-1 puis en centrifuger 110.10⁶. Retirer le surnageant et reprendre le culot avec les 60 mL de milieu supplémenté en PMA.
- Après homogénéisation répartir 5mL dans des puits de plaques 6 puits (5mL/puits) notés 1 à 21 = THP-1 activées.

### J1

Dans les plaques THP-1 activées (puits 1 à 21) : ôter le surnageant et répartir 5mL/puits de PBS-10%SVFi.

### J2

- Gratter chaque tapis cellulaire avec un cône bleu.
- Mettre la suspension dans un tube (flacon 15mL).
- Rincer deux fois les puits avec environ 2mL de **PBS** et ajouter à la suspension.
- Verser la totalité de la suspension sur 5 mL de Percoll (dilué au ½ dans du PBS) (flacon 15mL).
- Centrifuger 10 minutes à 1200 trs/min, accélération et décélération à 2, température à 20°C (SIGMA 3K15 ou MEGAFUGE 1.0R sans frein).
- Récupérer délicatement les cellules de l'anneau cellulaire dans un flacon 15mL.
- Compléter chaque tube à 15mL avec du PBS.
- Numérer la suspension cellulaire.
- Centrifuger la suspension à 1400 trs/min pendant 5 minutes, accélération et décélération à 7 (SIGMA 3K15 ou MEGAFUGE 1.0R avec frein) puis éliminer le surnageant.
Conserver les culots cellulaires à -20°C pour dosage du fer par ICP-SEA.

### III. Exemples de modèles Alzheimer appropriés permettant de confirmer l'efficacité des nanoparticules.

On utilise par exemple des modèles de souris transgéniques développant des plaques Aβ et des NFTs, et décrits dans :
- "Tau and transgenic animals models" J. Götz. Brain Research Reviews. (2001)
- "Animal models of cognitive dysfunction." S. K. Tayebati. Mech. of Ageing and Dev. (2006) ;
- "Transgenic mouse models of AD : how useful have they been for therapeutic development ?"K. Duff and S. Suleman. Briefing in Functional Genomics and Proteomics (2004) ;
- "Triple-transgenic model of AD with plaques and tangles : intracellular Aβ and synaptic dysfunction". S. Oddo et al. Neuron. (2003) ;
- le modèle APP Tg2576, Hsiao et al, Science, 1996 ;
- des modèles décrits dans J.Alzheimer.Dis, 9, 135-149, Mice models transgenic approaches, 2006, Games.

On utilise par exemple des modèles de rats transgéniques obtenus après incorporation de protéine Tau capable de générer les symptômes de la maladie d'Alzheimer.

On utilise par exemple des modèles de lapin utilisant l'injection d'aluminium-maltolate (agent neurotoxique) engendrant la formation d'agrégats de neurofilaments intraneuronaux Tau positifs, immunopositifs à Aβ, et décrits dans : « A new insight on Al-maltolate-treated aged rabbit as Alzheimer's animal model ». N. M. Bharathi et al. Brain Research Reviews. (2006).

On utilise par exemple des modèles de primates décrits dans :
- « Alzheimer's Aβ vaccination of Rhesus monkeys (Macaca mulatta)." S. Gandy et al. Mech. of Ageing and Dev. (2004) ;
- "Emerging prospects for the disease-modiiying treatment of AD". L. C. Walker et al. Bioch. Pharmacol. (2005) ;

### IV. Méthodes et résultats d'imagerie de la maladie d'Alzheimer avec des nanoparticules selon l'exemple I.

La dose de nanoparticules injectée en intraveineux est de l'ordre de 100 à 2000 µmole Fe/kg, selon l'espèce animale étudiée (par exemple souris, rats, singes).

Une imagerie pré-injection est réalisée comme contrôle. L'imagerie RMN des nanoparticules est réalisée entre 1h et 7 jours post injection selon la dose et l'espèce retenue. Le délai permet une captation des nanoparticules et une élimination de la contribution sanguine.

Les images sont réalisées sur des IRM de champs magnétiques statiques variant entre 1.5T (champ clinique) et 20T (champ pré clinique), en condition *in vivo* et/ou *ex vivo.*

L'exploitation des résultats IRM se fait par analyse du signal et/ou par comptage d'hyper ou d'hypo signaux, selon la technique RMN utilisée.

Les séquences RMN peuvent être des séquences d'écho de spin ou d'écho de gradient. La résolution spatiale peut être de très haute résolution spatiale (200 à 30 µm dans le plan) pour une visualisation directe des nanoparticules ou une résolution spatiale intermédiaire (1000 à 200µm) pour une visualisation indirecte, selon le type de séquence utilisé. L'épaisseur de coupe peut être entre 5 mm et 50µm selon l'espèce animale et la séquence utilisée. Une faible épaisseur de coupe permet d'avoir un volume partiel moindre, et une large épaisseur de coupe permet d'obtenir un effet de susceptibilité supérieur.
Les séquences d'imagerie RMN sont par exemples les séquences écho de gradient classiques, les séquences écho de spin, les séquences à contraste positif (IRON, GRASP, ONRES, etc.), les séquences quantitatives (cartographies T1, T2 T2*), les séquences utilisant la phase du signal (par exemple SWI, Susceptibility Weigthed Imaging), les séquences dérivées de la spectroscopie haute résolution (double quantum), les séquences avec modules préparatoires pour obtenir un meilleur contraste (par exemple un module d'inversion récupération, comme la séquence utilisant le *radiation dumping*).

La validation de la cible peut être réalisée par histologie, par marquage du Fer, de l'activité macrophagique ou microgliale ou par une autre technique d'imagerie comme la fluorescence.

Exemple de protocole utilisé :

### Animal :

Ce protocole est réalisé sur des souris double-transgéniques APP/PS1 âgées de 12 à 18 mois. La particule est injectée en i.v. à une dose comprise entre 100 et 1000 µmol de fer/kg. L'imagerie post-injection a été réalisée au moins 24h après l'injection.

### Imagerie :

Elle a été réalisée à 7T. La séquence d'acquisition est une écho de gradient 3D de 40 min et 12h respectivement en condition *in* et *ex vivo.* La résolution est de 50x50x(300-100) µm³. La même séquence a été appliquée avant et après l'injection.

## Revendications

1. Utilisation de nanoparticules métalliques comprenant un noyau recouvert d'une couche non polymérique dépourvue de biovecteur de ciblage, de formule (II) :
X-L-CH(PO₃H₂)₂,
dans laquelle
L représente un groupe organique de liaison reliant la fonction X à la fonction *gem-*bisphosphonate -CH(PO₃H₂)₂ choisi parmi :
- un groupe aliphatique, alicyclique ; alicyclique aliphatique ; aromatique ; aromatique aliphatique, lesdits groupes aliphatiques, alicycliques et aromatiques pouvant être éventuellement substitués par un groupe méthyle, hydroxy, méthoxy, acétoxy, amido, ou un atome d'halogène;
- un groupe -L₁-NHCO-L₂ où L₁ et L₂, identiques ou différents, représentent un groupe aliphatique ; alicyclique ; aromatique ; alicyclique aliphatique ou aromatique aliphatique, lesdits groupes pouvant être éventuellement substitués par un groupe méthyle, hydroxy, méthoxy, acétoxy, amido ou un atome d'halogène ;
et X représente un groupe COOH, -NH₂, -NCS, -NH-NH₂, -CHO, alkylpyrocarbonyle, acylazidyle, iminocarbonate, vinylsulfuryle, pyridyldisulfuryle, haloacétyle, maléimidyle, dichloro-triazinyle ou un atome d'halogène
couche sur laquelle sont greffés
a) le groupe favorisant la captation macrophagique :
de formule 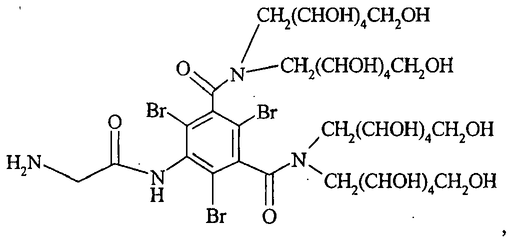 et/ou
b) des groupes polymériques favorisant le transfert de la BHE, choisis parmi des groupes PEG,
pour la préparation d'une composition pour le diagnostic en IRM, appliqué sur le corps humain ou animal, de la maladie d'Alzheimer.

2. Utilisation selon la revendication 1 **caractérisée en ce que** le noyau est monocristallin.

3. Utilisation selon l'une des revendications 1 à 2 **caractérisée en ce que** les nanoparticules sont incorporés dans un vecteur de transfert de la BHE.

4. Utilisation selon la revendication 3 **caractérisée en ce que** le vecteur de transfert de la BHE comprend un biovecteur de ciblage de l'AD, avantageusement un biovecteur de ciblage précoce de l'AD.

5. Utilisation selon l'une des revendications 3 à 4 **caractérisée en ce que** le vecteur de transfert de la BHE est apte à libérer les nanoparticules après activation physique ou chimique.

6. Utilisation selon l'une des revendications 3 à 5 **caractérisée en ce que** le vecteur de transfert de la BHE est un liposome ou un surfactant.

7. Utilisation selon l'une des revendications 3 à 6 **caractérisée en ce qu'**un agent thérapeutique contre l'AD est incorporé dans le vecteur de transfert de la BHE.

8. Utilisation selon l'une des revendications 1 à 7 **caractérisée en ce que** la composition diagnostic comprend en outre un agent facilitant le passage de la BHE.

9. Utilisation selon l'une des revendications 1 à 8 **caractérisée en ce que** l'imagerie IRM de la composition diagnostique est combinée à au moins une autre modalité d'imagerie, avantageusement l'imagerie optique, l'imagerie PET, le scanner aux rayons X, l'imagerie SPECT et/ou l'imagerie de fluorescence.

10. Utilisation selon l'une des revendications 1 à 9, **caractérisé en ce que** la composition est destinée au suivi diagnostic de l'efficacité d'un médicament ou d'un candidat médicament contre la maladie d'Alzheimer.

## Patentansprüche

1. Verwendung metallischer Nanopartikel, umfassend einen Kern, der mit einer nichtpolymeren Schicht ohne Ansteuerungsbiovektor der Formel (II) beschichtet ist:
X-L-CH(PO₃H₂)₂.
worin
L für eine organische Verbindungsgruppe steht, die die Funktion X mit der *gem*-Bisphosphonat-Funktion -CH(PO₃H₂)₂ verbindet, ausgewählt aus :
- einer aliphatischen, alicyclischen; alicyclischen aliphatischen; aromatischen; aromatischen aliphatischen Gruppe, wobei diese aliphatischen, alicyclischen und aromatischen Gruppen gegebenenfalls mit einer Methyl-, Hydroxy-, Methoxy-, Acetoxy-, Amidogruppe oder einem Halogenatom substituiert sein können,
- einer Gruppe -L₁-NHCO-L₂, wobei L₁ und L₂, die gleich oder verschieden sind, für eine aliphatische; alicyclische; aromatische; alicyclische aliphatische oder aromatische aliphatische Gruppe stehen, wobei diese Gruppen gegebenenfalls mit einer Methyl-, Hydroxy-, Methoxy-, Acetoxy-, Amidogruppe oder einem Halogenatom substituiert sein können,
und X für eine Gruppe COOH, -NH₂, -NCS, -NH-NH₂, -CHO, eine Alkylpyrocarbonyl-, Acylazidyl-, Iminocarbonat-, Vinylsulfuryl-, Pyridyldisulfuryl-, Halogenacetyl-, Maleimidyl-, Dichlortriazinylgruppe oder eine Halogenatom steht,
wobei auf die Schicht Folgendes gepropft ist:
a) die das Einfangen durch Makrophagen fördernde Gruppe der Formel: 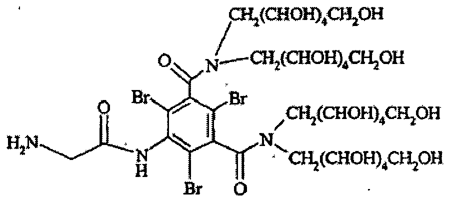 und/oder
b) polymere Gruppen, die den Transfer durch die BHS fördern, ausgewählt aus PEG-Gruppen,
zur Herstellung einer Zusammensetzung für die auf den menschlichen oder tierischen Körper angewendete MRT-Diagnose von Alzheimer-Krankheit.

2. Verwendung nach Anspruch 1, **dadurch gekennzeichnet, dass** der Kern monokristallin ist.

3. Verwendung nach einem der Ansprüche 1 bis 2, **dadurch gekennzeichnet, dass** die Nanopartikel in einen Vektor für den Transfer durch die BHS eingebracht sind.

4. Verwendung nach Anspruch 3, **dadurch gekennzeichnet, dass** der Vektor für den Transfer durch die BHS einen Ansteuerungsbiovektor für AD, vorteilhafterweise einen Biovektor für die frühzeitige Ansteuerung von AD, umfasst.

5. Verwendung nach einem der Ansprüche 3 bis 4, **dadurch gekennzeichnet, dass** der Vektor für den Transfer durch die BHS dafür ausgelegt ist, die Nanopartikel nach physikalischer oder chemischer Aktivierung freizusetzen.

6. Verwendung nach einem der Ansprüche 3 bis 5, **dadurch gekennzeichnet, dass** der Vektor für den Transfer durch die BHS ein Liposom oder ein oberflächenaktives Mittel ist.

7. Verwendung nach einem der Ansprüche 3 bis 6, **dadurch gekennzeichnet, dass** ein therapeutisches Mittel gegen AD in den Vektor für den Transfer durch die BHS eingebracht wird.

8. Verwendung nach einem der Ansprüche 1 bis 7, **dadurch gekennzeichnet, dass** die diagnostische Zusammensetzung außerdem ein Mittel umfasst, das den Übertritt über die BHS erleichtert.

9. Verwendung nach einem der Ansprüche 1 bis 8, **dadurch gekennzeichnet, dass** die MRT-Bildgebung der diagnostischen Zusammensetzung mit mindestens einer weiteren Bildgebungsart kombiniert wird, vorteilhafterweise mit optischer Bildgebung, PET-Bildgebung, Röntgenscanner, SPECT-Bildgebung und/oder Fluoreszenzbildgebung.

10. Verwendung nach einem der Ansprüche 1 bis 9, **dadurch gekennzeichnet, dass** die Zusammensetzung für die diagnostische Nachverfolgung der Wirksamkeit eines Arzneimittels oder eines Arzneimittelkandidaten gegen Alzheimerkrankheit bestimmt ist.

## Claims

1. Use of metal nanoparticles comprising a core covered with a nonpolymeric layer devoid of targeting biovector, of formula (II):
X-L-CH(PO₃H₂)₂
in which:
L represents an organic connecting group which links the functional group X to the gem-bisphosphonate functional group -CH(PO₃H₂)₂ chosen from:
- an aliphatic, alicyclic; alicyclic aliphatic; aromatic ; aromatic aliphatic group, it being possible for the said aliphatic, alicyclic and aromatic groups to be optionally substituted by a methyl, hydroxyl, methoxy, acetoxy or amido group or a halogen atom;
- an -L₁-NHCO-L₂ group, where L₁ and L₂, which are identical or different, represent an aliphatic; alicyclic; aromatic; alicyclic aliphatic or aromatic aliphatic group, it being possible for the said groups to be optionally substituted by a methyl, hydroxyl, methoxy, acetoxy or amido group or a halogen atom;
and X represents a COOH, -NH₂, -NCS, -NH-NH₂, -CHO, alkylpyrocarbonyl, acylazidyl, iminocarbonate, vinylsulphuryl, pyridyldisulphuryl, haloacetyl, maleimidyl or dichlorotriazinyl group or a halogen atom,
to which layer are grafted:
a) the group promoting macrophage uptake:
of formula 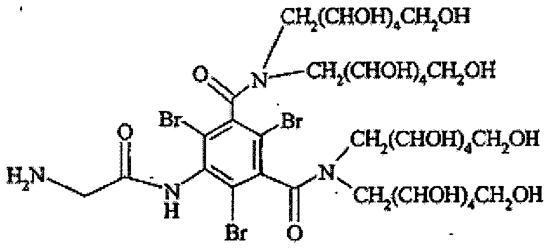 and/or
b) polymeric groups promoting BBB transfer, chosen from PEG groups,
for the preparation of a composition for the diagnosis by MRI, applied to the human or animal body, of Alzheimer's disease.

2. Use according to Claim 1, **characterized in that** the core is monocrystalline.

3. Use according to either of Claims 1 and 2, **characterized in that** the nanoparticles are incorporated in a BBB transfer vector.

4. Use according to Claim 3, **characterized in that** the BBB transfer vector comprises an AD-targeting biovector, advantageously a biovector for early targeting of AD.

5. Use according to either of Claims 3 and 4, **characterized in that** the BBB transfer vector is capable of releasing the nanoparticles after physical or chemical activation.

6. Use according to one of Claims 3 to 5, **characterized in that** the BBB transfer vector is a liposome or a surfactant.

7. Use according to one of Claims 3 to 6, **characterized in that** a therapeutic agent against AD is incorporated in the BBB transfer vector.

8. Use according to one of Claims 1 to 7, **characterized in that** the diagnostic composition additionally comprises an agent for facilitating passage across the BBB.

9. Use according to one of Claims 1 to 8, **characterized in that** the MRI imaging of the diagnostic composition is combined with at least one other imaging method, advantageously optical imaging, PET imaging, X-ray scanning, SPECT imaging and/or fluorescence imaging.

10. Use according to one of Claims 1 to 9, **characterized in that** the composition is intended for the diagnostic monitoring of the efficacy of a drug or a drug candidate against Alzheimer's disease.
